# EUROPEAN PATENT SPECIFICATION

(11) **EP 0 559 208 B1**
(45) Date of publication and mention of the grant of the patent: **06.09.2000**
(21) Application number: 93103556.2
(22) Date of filing: 05.03.1993
(51) Int. Cl.: G01N 15/14, G01N 33/569, G01N 33/50

(54) **Method for preparation and analysis of leukocytes in whole blood utilizing flow cytometry**
Verfahren zur Herstellung und Analyse von Leukozyten in Vollblut unter Verwendung der Durchflusszytometrie
Procédé de préparation et d'analyse de leucocytes dans le sang entier par cytométrie d'écoulement

(30) Priority: 05.03.1992 US 846316; 10.02.1993 US 15759
(43) Date of publication of application: 08.09.1993
(73) Proprietor: Becton, Dickinson and Company, Franklin Lakes, New Jersey 07417-1880 (US)
(72) Inventor: Jackson, Anne Louise, Ridgefield, Washington 98642 (US); Hoffman, Robert Alan, Livermore, California 94550 (US); Blidy, Andrew D., Redwood City, California 94063 (US); Murchison, Kenneth Earl, San José, California 95131 (US); Bierre, Pierre, Redwood City, California 94063 (US); Thiel, Dan E., Pleasanton, California 94566 (US)
(74) Representative: Rambelli, Paolo

(56) References cited:
- EP-A- 0 314 406
- WO-A-93/05478
- CYTOMETRY vol. 11, 1990, NEW YORK, NY pages 453 - 459 LOKEN ET AL. 'Establishing optimal lymphocyte gates for immunophenotyping by flow cytometry'
- JOURNAL OF IMMUNOLOGICAL METHODS vol. 123, 1989, AMSTERDAM pages 103 - 112 TERSTAPPEN ET AL. 'A rapid sample preparation technique for flow cytometric analysis of immunofluorescence allowing absolute enumeration of cell subpopulations'
- PROCEEDINGS OF THE NATIONAL ACADEMY OF SCIENCES vol. 83, no. 21, 1986, WASHINGTON, DC pages 8361 - 8365 HORAN ET AL. 'Improved flow cytometric analysis of leukocyte subsets: Simultaneous identification of five cell subsets using two-color immunofluorescence'
- CYTOMETRY vol. 12, 1991, NEW YORK, NY pages 445 - 454 BEGG ET AL. 'Cell kinetic analysis of mixed populations using three-color fluorescence flow cytometry'

## Description

### FIELD OF THE INVENTION

The present invention relates to a method for distinguishing and identifying leukocyte subpopulations in a blood or bone marrow sample. More particularly, the invention relates to methods for distinguishing and identifying subpopulations of leukocytes in a blood or bone marrow sample by flow cytometry using fluorescence and light scattering characteristics of the leukocytes.

### BACKGROUND OF THE INVENTION

In the field of hematology it is often useful to distinguish and identify the various types of white blood cells (leukocytes) which are present in the blood or bone marrow. Analysis of subpopulations of leukocytes is of particular interest for evaluation of immune system-related diseases such as acquired immune deficiency syndrome (AIDS). In particular, analysis of lymphocytes, a mononuclear leukocyte involved in the immune response, has clinical significance for management of immune system disorders. For example, it may be useful to detect or quantify the proportions of T-cell and B-cell subsets, the helper/inducer subsets of T-cells or the suppressor/cytotoxic subsets of T-cells. Monocytes, granulocytes (neutrophils), eosinophils and basophils are other leukocyte subpopulations of clinical interest in addition to lymphocytes. These cell types can be resolved and analyzed by flow cytometry.

Flow cytometers are useful for detecting particles such as cells. These instruments have means for detecting forward scattered and side scattered light as well as one or more means for detecting fluorescence. Forward and side light scatter are used to determine physical parameters, e.g. cell size and granularity. The various fluorescence detectors are used to distinguish cells labeled with fluorochromes which can be excited to emit light at different wavelengths. Examples of fluorochromes known in the art for use in flow cytometry are fluorescein isothiocyanate (FITC), phycoerythrin (PE), propidium iodide (PI) and peridinin chlorophyll protein (PerCP). All of these fluorochromes can be excited using light having a wavelength of 488 nm, but their emission wavelengths are different. FITC emits fluorescence at about 530 nm, PE and PI emit fluorescence at about 570 nm and PerCP emits fluorescence at about 670 nm, thereby permitting detection of three fluorescences of different colors using a single excitation wavelength.

To analyze a particular subpopulation of leukocytes by flow cytometry, the flow cytometer is set to trigger on a selected parameter. "Trigger" refers to a preset threshold for detection of a parameter. It is typically used as a means for detecting passage of a cell or other particle through the laser beam. Detection of an event which exceeds the threshold for the selected parameter triggers acquisition of light scatter and fluorescence data for the particle. Data is not acquired for cells or particles which cause a response below the threshold. The trigger parameter is typically the detection of forward scattered light caused by passage of a cell or particle through the light beam. The flow cytometer then detects and collects the light scatter and fluorescence data for the cell.

A particular subpopulation of interest's then further analyzed by "gating" based on the data collected for the entire population. To select an appropriate gate, the data is plotted so as to obtain the best separation of subpopulations possible. This is typically done by plotting forward light scatter (FSC) vs. side (i.e., orthogonal) light scatter (SSC) on a two dimensional dot plot. The flow cytometer operator then selects the desired subpopulation of cells (i.e., those cells within the gate) and excludes cells which are not within the gate. Typically, the operator selects the gate by drawing a line around the desired subpopulation using a cursor on a computer screen. Only those cells within the gate are then further analyzed by plotting the other parameters for these cells, such as fluorescence. While gating is a powerful tool for removing data for cells which are not of interest and thereby improving the ability to analyze the desired subpopulation, it can be a source of substantial error if separation of the subpopulations is not optimal.

Resolution of leukocyte subpopulations by light scattering is impaired when unlysed or whole blood samples are analyzed by flow cytometry. This is due to the fact that red blood cells (erythrocytes) present in the sample interfere with light scatter measurements and obscure signals from other cell types. Interference is particularly large for forward scatter, causing large errors in analysis of lymphocytes. For this reason, practitioners typically lyse the red blood cells in a sample when it is desired to analyze the leukocytes. Several reagents for lysing erythrocytes which are useful in the invention (e.g., water, saponin and the other lysing reagents discussed below) are known in the art. The most commonly used is buffered ammonium chloride. Although generally adequate separation of debris from lymphocytes using scatter gating in ammonium chloride solutions can be obtained, the absence of a fixative means the biological sample may present a biohazard. Addition of a fixative such as paraformaldehyde to ammonium chloride lysing solutions is unsatisfactory, however, due to generation of hydrogen ions which lower the pH of the sample and reduce fluorescence from fluorescein, thereby impairing immunofluorescence analysis.

Lysing reagents comprising formaldehyde, a salt of a weak acid and a polyhydric alcohol are also known in the art (U. S. Patent Nos. 4,902,613 and 4,654,312). This lysing reagent is available from Becton Dickinson Immunocytometry Systems, San Jose, California under the name FACS Lysing Solution and comprises diethylene glycol, heparin, citrate buffer and formaldehyde, pH 7.2. Because of the presence of formaldehyde, FACS Lysing Solution provides the advantage of reducing the biohazard from biological samples. However, its presence in a sample being analyzed by flow cytometry causes cell shrinkage and may result in unsatisfactory levels of debris which can obscure the cell population to be gated, particularly lymphocytes. Other lysing reagents known in the art are described below in connection with "no-wash" sample preparation methods.

To reduce the negative effect of lysing reagents on light scattering parameters the lysed sample is typically centrifuged and washed to remove debris, unbound fluorochrome-labeled antibodies and the lysing solution itself, all of which contribute to poor light scatter analysis. While this method partially solves the problem of resolving leukocyte subpopulations based on light scattering characteristics, it is labor intensive, adds steps to the procedure, is difficult to automate and can result in a loss of cells which precludes making accurate cell counts on the sample. For example, to obtain accurate CD4+ lymphocyte counts from lysed, washed blood samples, presently available methods require multiple steps and complex data analysis.

It is therefore desirable to have methods available for preparation of lysed blood samples which do not require removal of the lysing solution and washing prior to flow cytometric analysis. Such "no-wash" sample preparation methods for flow cytometry are known in the art. Caldwell, et. al (AJCP 86:600-607 (1986)) describe a no-wash method for immunophenotypic analysis of cells by flow cytometry in which lymphocytes are isolated from peripheral blood samples by fractionation on density gradients, obviating the need for erythrocyte lysis. After immunofluorescent staining, samples are subjected to flow cytometric analysis without washing, leaving unbound fluorescent label in the analyzed sample. Lymphocytes are identified and gated using forward and side light scatter parameters.

U.S. Patent No. 5,030,554 discloses treatment of a blood sample with a lytic agent comprising a water soluble, organic carboxylic acid having a pK value greater than 3.0, a pH of about 2.6 to 4.0 and a counterion which does not materially alter the ionic strength of the sample. The separate staining, lysing and quenching steps are performed in a single vessel without the need for removal of unreacted stain and/or unconsumed reagents from the sample prior to analysis. The leukocyte subpopulations are identified and resolved in the traditional manner, using forward light scatter and side light scatter parameters. This no-wash method is commercially available from Coulter Corporation under the name Q-PREP. Ortho Diagnostic Systems has also published a no-wash sample preparation protocol using ammonium chloride which notes that if analysis of the cells in the lysing solution is not to be performed within 2 hours, the cells should be centrifuged, washed, resuspended in phosphate buffered saline and analyzed within 8 hours.

WO 88/07187 discloses a reagent for lysis of red blood cells which comprises a low molecular weight carboxylic acid, a sulfonic acid or an activated phenol. The lysed sample is directly analyzed by flow cytometry to identify leukocyte subpopulations based on forward and angular light scatter. It is taught that red cell fragments do not interfere with or adversely effect the photometric differentiation of leukocyte subpopulations using this lysing reagent.

Several methods for analyzing and gating leukocyte subpopulations are known in the art. U.S. Patent No. 4,284,412 discloses an apparatus and method for gating a leukocyte subpopulation such as lymphocytes. A flow cytometer is modified such that one of the right angle-fluorescence channels serves as a wavelength specific sensor for right angle scatter. A lymphocyte subclass of interest is fluorescently labeled and the sample is analyzed, using detection of forward and right angle scatter pulses within specified ranges to actuate acquisition of a fluorescent signal, thereby gating the lymphocyte population. The apparatus and method are used in conjunction with lysis and washing of erythrocytes in the blood sample according to conventional procedures.

U. S. Patent No. 4,727,020 discloses a method for analysis of subpopulations of blood cells using a fluorescence trigger and either lysed and washed or unlysed samples. The antibodies for use in triggering include anti-HLe-1 and the acquired data are analyzed using two fluorescence parameters to resolve the subpopulations.

Terstappen, et al. (J. Immunol. Mtds. 123:103-112(1989)) describe a no wash method for flow cytometry sample preparation in which peripheral blood cells are labeled with the nucleic acid stain LDS-751. The red blood cells in the labeled sample are lysed in water prior to measurement on a flow cytometer. Immunofluorescence resolution of leukocytes and percentage of leukocyte subpopulations correlated well with results obtained with a clinical hematology analyzer and the nucleic acid stain allowed discrimination of intact cells from ghosts, platelets and damaged nucleated cells. The no-wash, hypotonic lysis procedure provided a means of obtaining absolute numbers of leukocyte subpopulations, whereas traditional lysis procedures which included centrifugation steps exhibited significant loss of cells and/or selective loss of lymphocyte subpopulations.

Gating methods which are not based solely on analysis of forward and side light scatter parameters are also known in the art. Hoffmann (Cytometry Supp. 1, pg. 36, Abstract No. 172 (1987)) describes the use of fluorescence to distinguish leukocyte subpopulations in unlysed blood samples, triggering on a fluorescence parameter and using light scatter to further differentiate leukocyte subpopulations. However, coincidence of erythrocytes with leukocytes partially obscured the right angle scatter distributions, providing suboptimal gating of lymphocytes. U. S. Patent No. 4,987,086 discloses a method for determination of leukocyte subpopulations utilizing flow cytometry in which a gate is established by 1) labeling with a fluorochrome-labeled antibody which reacts with all leukocytes, and 2) labeling monocytes with a fluorochrome-labeled antibody specific for monocytes. This method is also taught by Loken, et al. (Cytometry 11:453-459 (1990)). To establish the gate, the cells are analyzed for forward light scatter and side light scatter as well as the two colors of fluorescence associated with the antibodies. A computer algorithtm compares the light scatter data with the fluorescence analysis and differentiates those cells which should be included within the gate. The computer controlled gating analysis eliminates much of the subjectivity and error associated with visual gating methods. However, although the accuracy of the gating procedure is improved, this method has the disadvantage of requiring separate preparation of at least two sample aliquots and at least two separate flow cytometer runs for analysis of each sample (i.e., the gating tube and the experimental tube). This gating protocol is sold by Becton Dickinson Immunocytometry Systems under the name LEUCOGATE.

Horan, et al. (PNAS 83:8361-8365 (1986)) describe a method for analysis of leukocyte subsets in which five cell subsets are simultaneously identified using two-color immunofluorescence. The identification of subsets can be made with fluorochrome labeling of only a single aliquot of a sample, in contrast to prior art methods in which multiple aliquots of the same sample must be stained. Horan, et al. use dilutions of the fluorochrome-labeled antibody reagents to produce discrete fluorescence intensity profiles for cell subsets that would otherwise overlap or be indistinguishable when stained with antibodies bearing the same fluorochrome.

The present invention as defined in claim 1 overcomes these problems in the art. It provides a method for establishing flow cytometry gates for leukocyte subpopulations under conditions where forward light scatter and side light scatter alone are unsatisfactory for this purpose.

The invention as defined in claim 1 also provides a method for preparing a lysed blood sample for analysis of leukocytes by flow cytometry which does not involve centrifugation and washing of the stained and lysed sample prior to analysis on a flow cytometer. In the clinical laboratory setting, results are generally rejected for samples with less than 90% lymphocytes included in the gate. Using the inventive methods to trigger out debris, including much greater than 90% lymphocytes in the gate is more easily achieved. In addition, the inventive methods can be used to correct data from all samples with less than 90% lymphocytes gated when debris or nonlymphocyte cells are the source of the gating problem.

### SUMMARY OF THE INVENTION

A method of flow cytometric analysis of leukocyte sub-populations is provided in which, in a single aliquot of the sample, the leukocytes are labelled with a leukocyte-specific fluorochrome-labelled primary antibody which binds to different extents to the cells of the sub-population of interest and to the cells of at least to one additional sub-population with different fluorescence intensities; red blood cells in the sample are lysed by addition of a lysing reagent; the labelled leukocytes are analysed in the lysed sample on a flow cytometer without separation of the labelled leukocytes from the lysing reagent, the cytometer being set with a triggering threshold representative of a predetermined fluorescence intensity indicative of the presence of a primary antibody, such that all cells for which said threshold is exceeded, are analysed. Said analysing comprises acquiring fluorescence and light scatter data for the labelled leukocytes, and identifying the sub-population of cells of interest by a method including generating a first plot of a light scatter intensity parameter vs. antibody fluorescence intensity and selecting a gate area of said plot as said sub-population of cells of interest.

### DESCRIPTION OF THE DRAWINGS

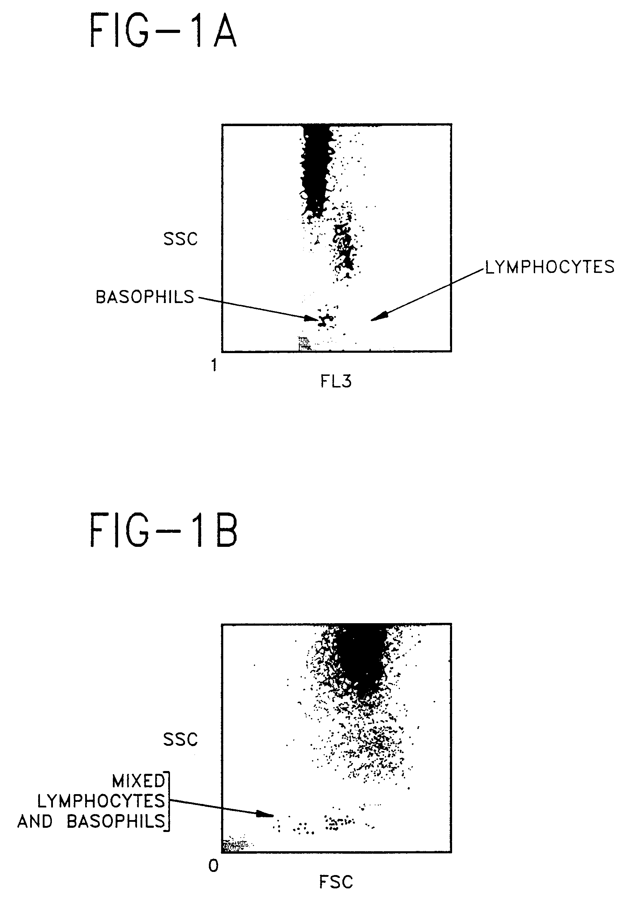

Fig.1 illustrates the ability of the inventive method to resolve basophils from lymphocytes (Fig 1A), whereas conventional scatter gating does not (Fig. 1B).

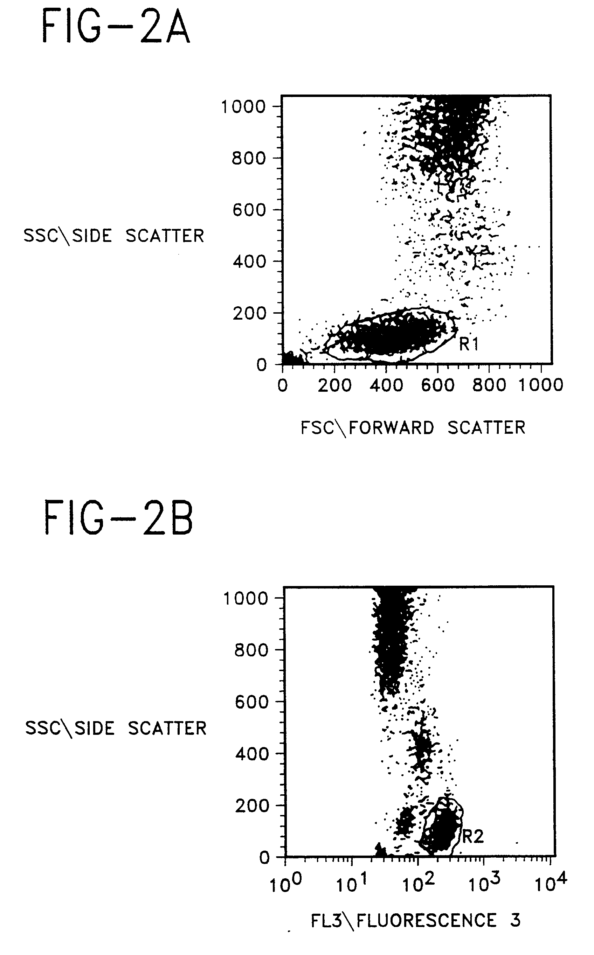

Fig.2 illustrates 2-parameter gating according to the invention. Conventional FSC/SSC scatter gating (R1, Fig. 2A) is compared to the 2-parameter gating method of the invention (R2, Fig. 2B). Figs. 2C and 2D are plots of log fluorescence (FL1, anti-Leu-M7-FITC vs. log fluorescence (FL2, anti-Leu-M3-PE) for the gating methods of Fig. 2A and Fig. 2B, respectively, showing the amount of non-lymphocytic contamination included in each gate.

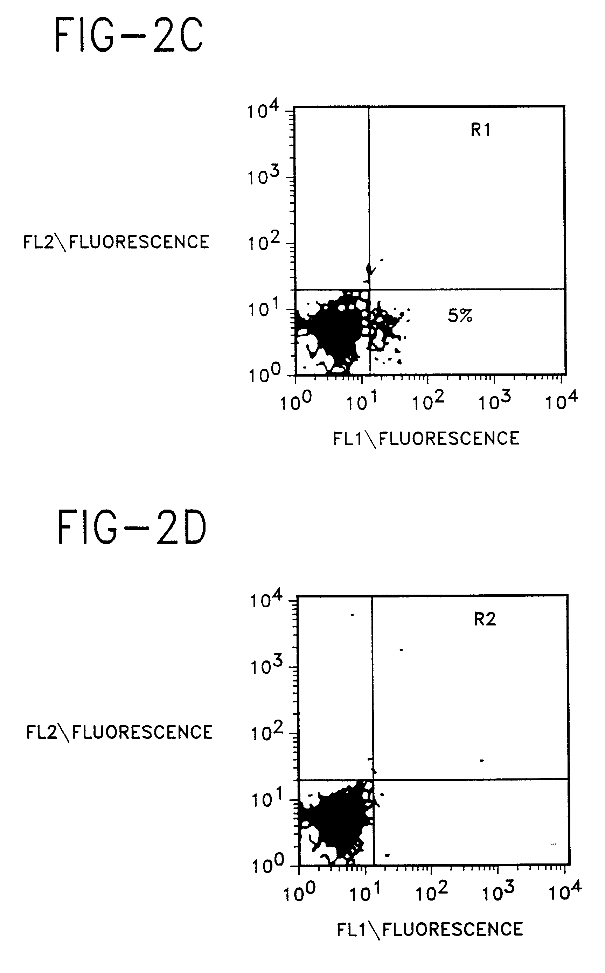

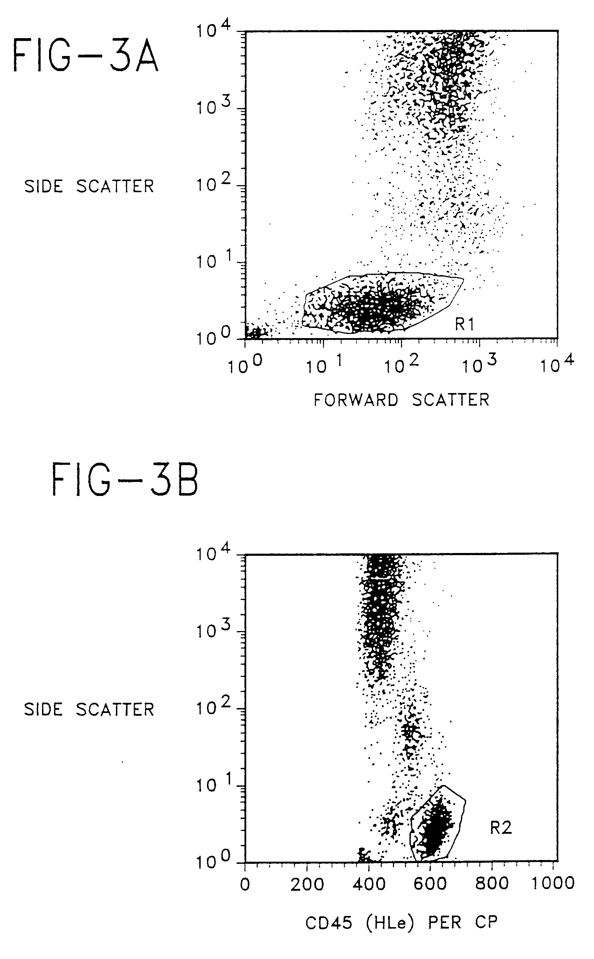

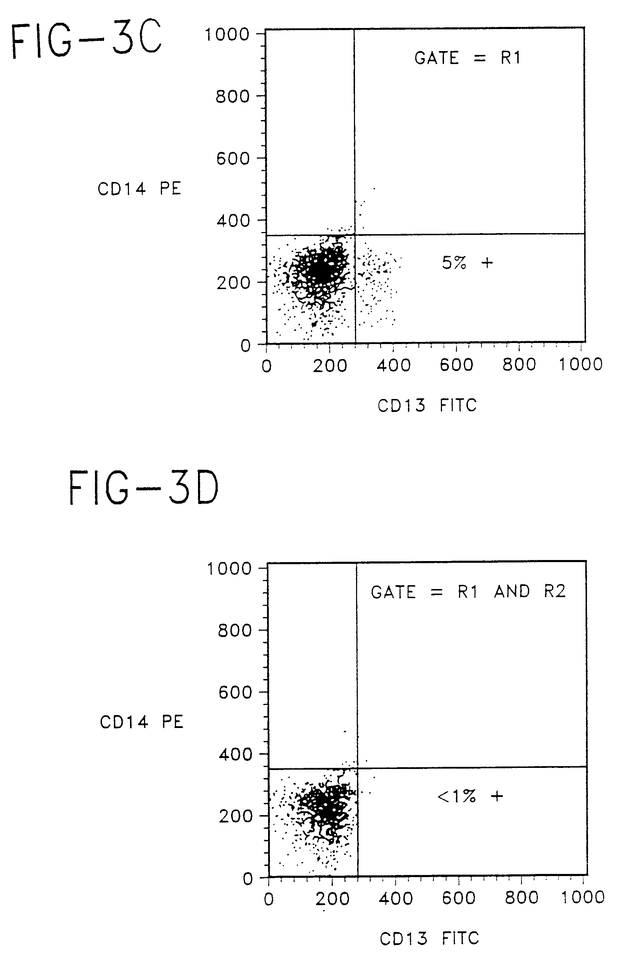

Fig. 3 illustrates 3-parameter gating according to the invention. Fig. 3A shows the R1 lymphocyte gate established by analysis of SSC/FSC parameters. Fig. 3B shows the R2 lymphocyte gate established by analysis of SSC/FL3 parameters. Fig. 3C shows an analysis of the FL1 and FL2 parameters of the R1-gated lymphocytes, demonstrating the myeloid nature of the contaminating non-lymphocytic cells. Fig. 3D shows an analysis of the FL1 and FL2 parameters of the (R1+R2) (i.e., 3-parameter) gated lymphocytes, demonstrating the reduction in contaminating myeloid cells by this method.

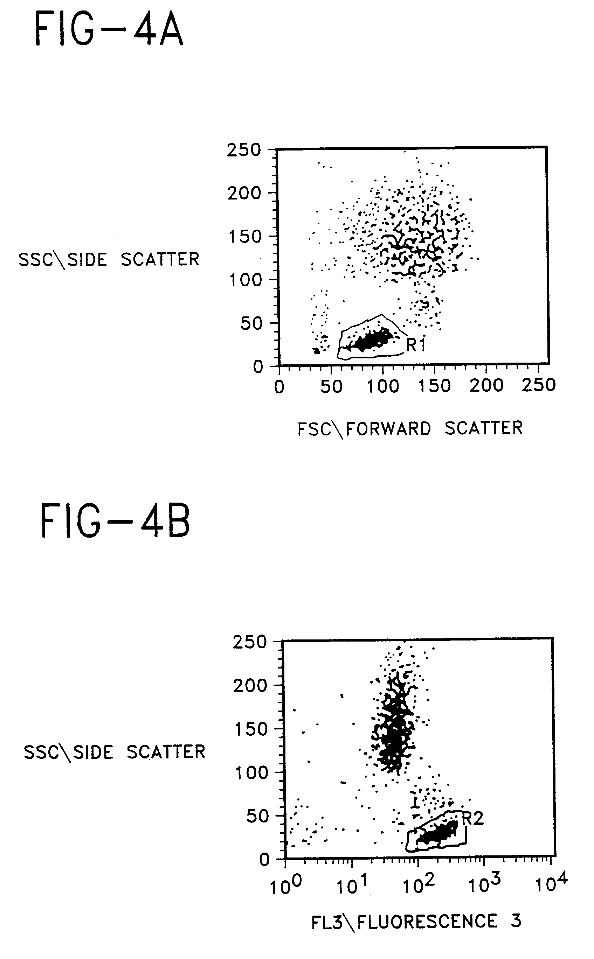

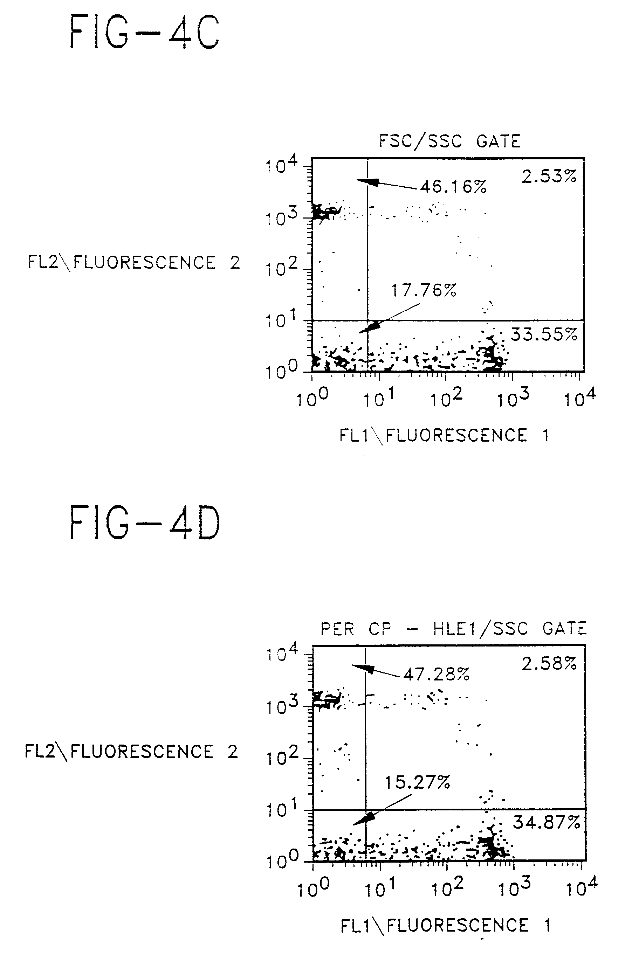

Fig. 4 illustrates the ability of the gating method of the invention to provide accurate cell counts even when lysed blood samples contain residual red blood cells. Fig. 4A shows cell counts obtained by analysis of lysed, washed blood samples gated on SSC/FSC. Fig. 4B shows cell counts obtained by analysis of lysed, washed blood samples gated on SSC/FL3. Fig. 4C shows cell counts obtained by analysis of lysed, washed blood samples containing residual erythrocytes gated on SSC/FSC. Fig. 4D shows cell counts obtained by analysis of lysed, washed blood samples containing residual erythrocytes gated on SSC/FL3.

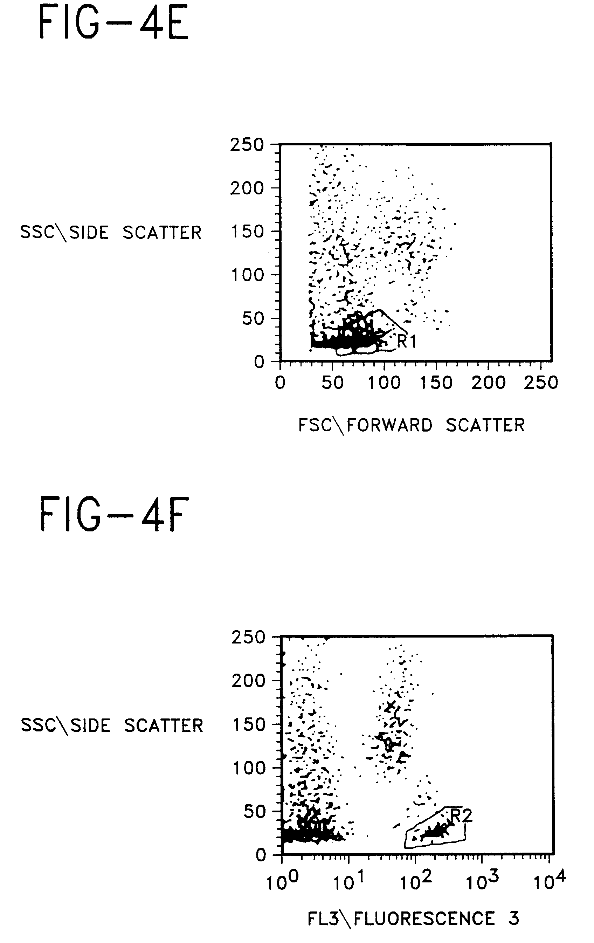

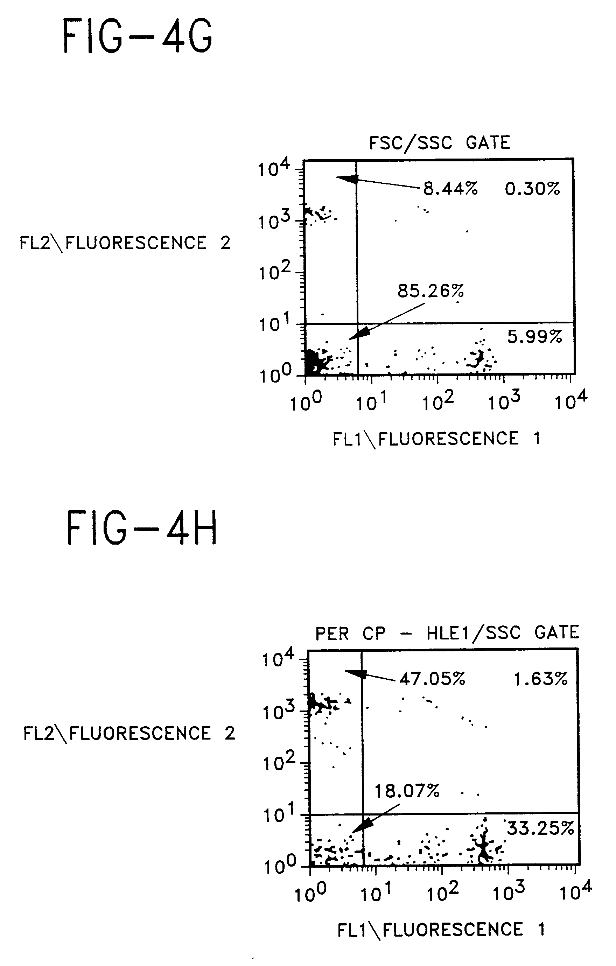

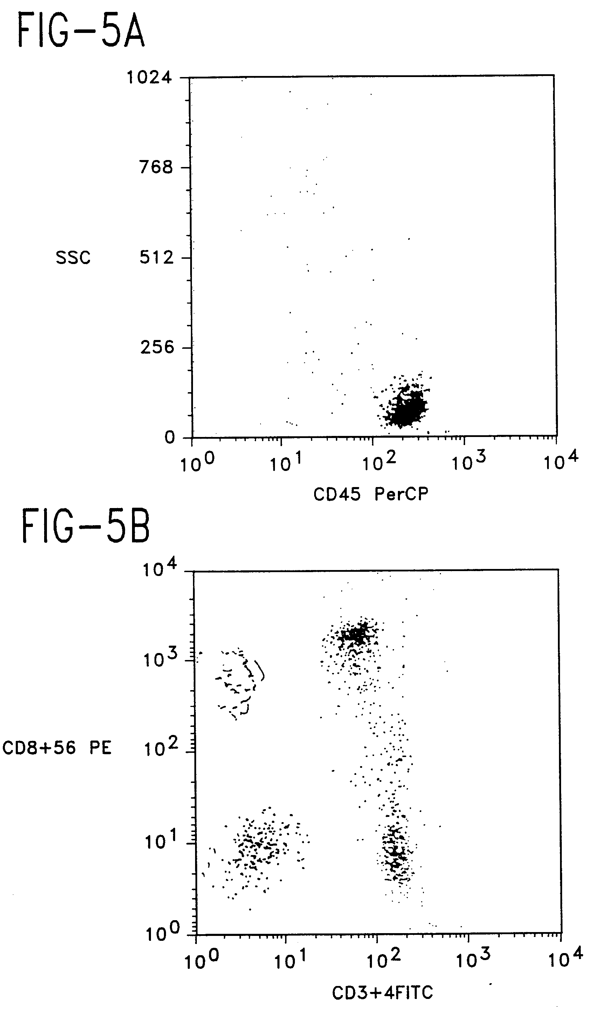

Fig. 5 illustrates a three-part differential blood count (T, B and NK cells) with CD4/CD8 T-cell subsetting using the lyse, no-wash methodology of the invention with cluster analysis performed using attractors software. Fig. 5A shows the classified lymphocytes after attractors analysis of the listmode data. Fig. 5B shows the four lymphocyte subpopulations identified by fluorescence.

### DETAILED DESCRIPTION OF THE INVENTION

The present invention provides a method using flow cytometry for identifying and enumerating cells representing subpopulations of leukocytes in a biological sample containing leukocytes mixed with other blood cell types (e.g., blood or bone marrow samples). The method utilizes a fluorescence trigger to exclude selected subpopulations of blood particles prior to gating, and a gate established by analysis of acquired data parameters to exclude selected subpopulations of leukocytes from data analyses. A 2-parameter gate may be established based on analysis of a plot of a single light scattering parameter vs. a single fluorescence parameter (e.g., SSC and fluorescence). Alternatively, a 3-parameter gate may be established based on a combined analysis of SSC, FSC and fluorescence parameters, including such a plot.

For this purpose, a fluorochrome-labeled antibody which recognizes an antigen or marker associated with all leukocyte subpopulations (the "primary antibody") is used to stain the cells of the sample. The primary antibody is selected so that it binds to all leukocyte cell types but preferably with a detectably different intensity on each subpopulation. That is, the primary antibody is directed against a leukocyte antigen which is present in different amounts on each leukocyte subpopulation. The primary antibody does not bind to any significant degree to debris or to cell types other than leukocytes. The intensity of fluorescence characteristic of each leukocyte subpopulation, in combination with other measured parameters, therefore allows differentiation of the leukocyte subpopulations by flow cytometry. The primary antibody, because it binds substantially only to leukocytes, also allows discrimination between leukocytes and red cells, debris such as cell membranes (ghosts), platelets and other particles in the sample which exhibit minimal binding of the primary antibody.

After staining the sample with the primary antibody, and optionally with at least one secondary antibody as described below, it is analyzed on a flow cytometer, preferably without centrifugation or washing of the cells. The flow cytometer is preferably set with a trigger threshold on fluorescence emitted by the fluorochrome on the primary antibody. Light scatter and antibody fluorescence data are collected for each cell detected. A dot plot or histogram of the data is generated using primary antibody fluorescence (PAbF) and one of the light scatter parameters (FSC or SSC). The leukocyte subpopulation of interest is then identified on the plot and gated using methods known in the art.

Establishing the gate using scatter vs. PAbF (a 2-parameter gate) provides significantly improved resolution of the leukocyte subpopulations from platelets, debris, etc. It is also possible by this method to discriminate between lymphocytes and basophils which are usually inseparable from lymphocytes when gating by conventional methods of data analysis (Fig. 1). The inventive method therefore provides a means for eliminating basophil contamination from the lymphocyte gate using a 2-parameter light scatter/fluorescence gate, improving the purity of the gated lymphocytes.

Alternatively, a 3-parameter gate may be established for the acquired data to improve resolution of the various leukocyte subpopulations. Typically, a first gate (R1) is established by analysis of FSC vs. SSC and a second gate (R2) is established by analysis of SSC vs. PAbF. When plotted and analyzed in two dimensions the 3-parameter gate (R3) is based on an analysis of R1 and R2 to select only the cells contained in both gates. In general, a whole blood sample is incubated with at least two fluorochrome-labeled antibodies. One of these is the primary antibody described above. One or more additional fluorochrome-labeled antibodies (the "secondary antibodies"), specific for an antigen or cell type of interest, may also be included in the staining mixture to provide additional fluorescent signals for analysis. The fluorochromes on the secondary antibodies emit fluorescent light at wavelengths which are separately detectable and can be distinguished from each other and from primary antibody fluorescence by the flow cytometer. The primary and secondary antibodies may recognize and bind to the same or different antigens.

The fluorochromes useful for conjugation to antibodies according to the present invention may be any of the fluorochromes commonly used in flow cytometry which can be conjugated to proteins such as antibodies. Fluorescein isothiocyanate (FITC), phycoerythrin (PE), and peridinin chlorophyll protein (PerCP) are preferred because they can be excited by light of the same wavelength but emit light at separately detectable wavelengths compatible with flow cytometric analysis. Any of these fluorochromes can be used to label the primary and secondary antibodies of the invention using conjugation methods well known in the art. See, for example, U. S. Patent No. 4,876,190 and references cited therein for a discussion of covalent and noncovalent conjugation of fluorochromes to proteins such as antibodies. The disclosure of U.S. 4,876,190 is incorporated herein by reference.

The primary and secondary antibodies may be polyclonal or monoclonal, but are preferably monoclonal antibodies. The primary antibody may be any antibody capable of binding to the entire cell population of interest with minimal binding to other cell types. Preferably leukocytes are bound by the primary antibody. Such anti-leukocyte monoclonal antibodies are known in the art and include, but are not limited to, CD45 (e.g., anti-HLe-1) and CD11a/CD18 (e.g., anti-LFA-1). The fluorochrome label on the primary antibody is not critical but is preferably FITC, PE or PerCP. CD45 conjugated to PerCP, which will bind to and identify leukocytes, is the most preferred primary antibody for use in the invention. CD45 has the advantage of being able to bind to fixed cells, allowing staining after lysis and fixation if desired, for example when the method of the invention is used to correct a previous gate which gave unsatisfactory results.

Secondary antibodies, labeled with a fluorochrome different from the fluorochrome on the primary antibody, may also be either polyclonal or monoclonal antibodies. Monoclonal antibodies are preferred for their enhanced specificity. The secondary antibodies are selected to be specific for and to bind to any antigen for detection of any cellular characteristic of interest to the investigator. Secondary antibodies may be used to identify either the gated subpopulation of cells (e.g., lymphocytes) or a subset of the subpopulation (e.g., T cells or B cells). If desired, the secondary antibody may comprise the same antibody used to identify the subpopulations (i.e., the antibody component of the primary antibody) but bound to a different fluorochrome than is used for the primary antibody so that it can be separately detected. For example, CD4 (e.g., anti-Leu-3a) may be used as secondary antibodies to detect helper/inducer T lymphocytes, CD8 (e.g., anti-Leu-2a) may be used to detect cytotoxic/suppressor T lymphocytes, CD14 (e.g., anti-Leu-M3) may be used to detect monocytes, CD13 (e.g., anti-Leu-M7) may be used to detect myeloid cells, CD3 (e.g., anti-Leu-4) may be used to detect T lymphocytes, and CD19 (e.g., anti-Leu-12) may be used to detect B lymphocytes. All of these antibodies as well as CD45 are available from Becton Dickinson Immunocytometry Systems, San Jose, California. CD56 (e.g., anti-Leu-19 or anti-NCAM), CD57 (e.g., anti-Leu-7), CD38 (e.g., anti-Leu-17), CD28 and anti-HLA-DR are also useful as components of secondary antibodies for certain applications in the practice of the invention. In addition, CD14 and CD13 can be used as quality control agents to assess the purity of the gated lymphocytes.

Other antibodies may be useful for detecting leukemic cells in blood samples according to the invention. For example, leukocytes may be stained using a primary antibody comprising a CD45 antibody and a secondary antibody comprising an antibody specific for leukemic cells of interest. Alternatively, a blood sample may be analyzed as described above, and if the initial results suggest an abnormality, the sample may be restained with secondary antibody which will bind to and specifically detect leukemic cells.

The particular fluorochrome selected as a component of the secondary antibody is not critical but is preferably FITC, PE, or PerCP. PE and/or FITC are most preferred for labeling the secondary antibody when PerCP is the fluorochrome on the primary antibody.

To label cells in the sample by binding to the primary and/or secondary antibodies, a sample such as whole blood or bone marrow is mixed with the fluorochrome-labeled primary and secondary antibodies. The labeled antibodies are generally added to the sample substantially simultaneously and mixed therewith. Cell labeling methods suitable for use in the invention are well-known in the art. For flow cytometry, the blood or bone marrow sample will generally be about 100 µl in volume and about 0.05 - 1.0 µg of each antibody will be added per sample. After addition of the desired labeled antibodies, the sample is incubated at room temperature, preferably in the dark, for about 5 - 30 min. For most applications, an incubation period of about 15 min. is generally sufficient to obtain satisfactory binding. It is generally possible to use less secondary antibody in the staining procedures of the invention (up to about a 20-fold reduction) than is typical for samples which are to be washed, because bound antibody is not washed off.

Lysing reagent may be added to the sample with the labeled antibodies or lysis may be performed prior to labeling. However, labeling the cells first with subsequent lysis of erythrocytes is preferred to maximizing binding of the antibodies. The sample is incubated in the presence of lysing reagent for about 10 min. Suitable lysing reagents for this purpose may include, but are not limited to, ammonium chloride and FACS Lysing Solution.

FACS Lysing Solution is preferred because it provides better lymphocyte/monocyte separation on side scatter than does ammonium chloride, provides stable light scatter and fluorescence results for extended periods of time, and allows lysing and fixing to be performed in a single step. A sample of stained, unwashed blood in FACS Lysing Solution generally remains stable for up to 48 hrs., and in some instances the light scatter even improves with increasing incubation time. In addition, the relatively high concentration of formaldehyde (3%) in FACS Lysing Solution may provide a higher degree of biosafety than other lysing reagents.

Because the presence of some erythrocytes in the sample does not significantly interfere with the gating method of the invention, for purposes of gating and flow cytometric analysis it is not necessary that all erythrocytes in the sample be lysed. It is therefore possible to use less lysing reagent than is customary for lysing methods which require that the sample be centrifuged and washed. For example, 2 ml of FACS Lysing Solution is generally added to a 100 µl blood sample. For use with the present methods, 0.5 - 1.0 ml of FACSTM Lysing Solution in 100 µl of blood gives satisfactory results on gating and flow cytometric analysis. Because there is reduced interference from red blood cells the invention will also provide better results for samples which contain erythrocytes which do not readily lyse under standard conditions (e.g., samples containing nucleated red blood cells).

The fluorochrome-labeled sample is analyzed on a flow cytometer. If the sample is lysed prior to analysis, it is analyzed in the lysing reagent without removal of the lysing reagent, centrifugation or washing. Any of the commercially available flow cytometers which are capable of light scatter and at least two-color fluorescence data acquisition may be used for this purpose. Flow cytometers which are capable of acquiring at least three-color fluorescence data are preferred because after triggering and gating using one fluorescence parameter according to the invention, two-color analysis of the cells of interest can still be performed. One example of such an instrument is the FACSCAN available from Becton Dickinson Immunocytometry Systems. As the inventive method is also applicable to analyses involving detection of four or more fluorescence parameters, instruments such as the FACSTAR PLUS may also be used. This flow cytometer has two lasers which produce light of different wavelengths and is capable of detecting five fluorescence parameters and two light scatter parameters. Such an instrument provides a particular advantage for use in the invention as it allows use of a fluorescence parameter for triggering and gating while retaining the capability to perform three (or more)-color fluorescence analysis of the cells of interest.

The flow cytometer should produce light of an appropriate wavelength for excitation of the selected fluorochromes. Acquisition of FSC, SSC, and all fluorescence data for the cells in the sample is triggered by fluorescence of the fluorochrome selected for labeling the primary antibody. Triggering on the bright PAbF events serves to exclude the majority of debris and platelet events as well as other events which are not due to leukocytes. All of these non-leukocyte events can interfere with FSC resolution if they are not excluded. The acquired data is stored electronically and converted by computer using an appropriate algorithm to data which can be plotted in the form of a dot plot for gating.

For two-parameter gating, the data plot is generated using light scatter vs. primary antibody fluorescence. This method is illustrated in Fig. 2. Most preferably the light scatter parameter is side scatter (SSC), but forward scatter (FSC) data may also be used. Primary antibody fluorescence is preferred (PAbF) as the second parameter. The scatter vs. fluorescence dot plot is used to gate the desired cell subpopulation. Gating may be accomplished by any of the art-recognized methods using algorithms compatible with the flow cytometer used for sample analysis. For example, SIMULSET, LYSYS or PAINT-A-GATE data analysis software (Becton Dickinson Immunocytometry Systems) can be used to gate and analyze data acquired on the FACSCAN flow cytometer. Once the acquired data is gated, light scatter and fluorescence data acquired for the gated subpopulation can be analyzed on dot plots, histograms, contour plots or by other means known in the art.

Alternatively, a 3-parameter gate maybe established for the acquired data to further improve resolution of the various leukocyte subpopulations and eliminate contamination from the gate. This method is illustrated in Fig. 3. R1 may be established first by analysis of SSC and FSC with R2 established subsequently by analysis of SSC and PAbF. However, R1 and R2 may be reversed, establishing R1 first by analysis of SSC and PAbF and R2 second by SSC and FSC. A computer algorithm then compares R1 and R2 and establishes R3 to include the cells contained in both the R1 and R2 gates. The 3-parameter gating of the present invention, with appropriate software, also allows three-dimensional plotting and analysis of the three parameters. Such three-dimensional plotting and/or gating significantly improves resolution of all five leukocyte subpopulations (lymphocytes, monocytes, neutrophils, basophils and eosinophils) and therefore can allow calculation of a five part differential blood count using flow cytometry. Although currently available single-threshold instruments can perform such analyses, a flow cytometry instrument with dual-threshold capability would be more desirable for this purpose, as it would reduce debris and the number of cells that would have to be analyzed to obtain a reliable result.

Addition of the primary antibody of the invention to gating procedures known in the art, such as LEUCOGATE, with triggering on FL3 significantly reduces acquisition of debris in lysed unwashed blood samples and improves resolution of leukocyte subpopulations. This improvement is possible without reducing the number of fluorescence parameters which are available for data acquisition and analysis since trigger fluorescence need not be stored and treated as data. This is a particular advantage when using software in which the number of fluorescence parameters which can be analyzed is limited. For example, a lysed unwashed aliquot of a sample to be analyzed can be stained or labeled according to the standard LEUCOGATE procedure using an anti-leukocyte antibody conjugated to a first fluorochrome and an anti-monocyte antibody conjugated to a separately detectable second fluorochrome (e.g., anti-HLe-1-FITC and anti-Leu-M3-PE). The aliquot is also labeled with an anti-leukocyte primary antibody according to the invention comprising a third fluorochrome which can be differentiated from the first and second fluorochromes (e.g., anti-HLe-1-PerCP). The aliquot is then run on a flow cytometer set to trigger on FL3 (PerCP).

If it is desired to have available all of the fluorescence parameter analysis capability of the software, FL3 data are not stored. For example, SIMULSET software is limited to analysis of two fluorescence parameters and two light scatter parameters. To avoid limiting the system to analysis of a single fluorescence parameter in addition to the trigger, FL3 data (i.e., the trigger) would not be stored, leaving two fluorescence parameters available for data analysis. Data for the remaining two fluorescence parameters (FL1 and FL2) and the light scatter parameters are acquired and stored for particles exceeding the threshold and the gate is established by the software. This is the conventional SSC/FSC scatter gate when using SIMULSET. The gate established by this procedure is then applied to data obtained for a test aliquot of the sample labeled with the same primary antibody for triggering and any additional antibodies desired for flow cytometric analysis.

In the single-tube analysis format of the invention, the method provides a means for obtaining a maximum amount of information with minimal sample manipulation, resulting in time savings both in sample preparation and instrument data acquisition. It is therefore particularly suited for use in clinical laboratories which require high-throughput diagnostic products for tests such as routine lymphocyte enumeration. In a preferred embodiment, the present no-wash analysis method is used to obtain a three-part differential blood count (T, B and NK cells) with T-cell subsetting into CD4 (helper) and CD8 (suppressor) in a single tube flow cytometric analysis. The method allows detection of two or more subpopulations of leukocytes for each chromophore, for example using FITC and PE two-color fluorescence on a FACScan.

For example, for a three-part differential analysis with CD4/CD8 subsetting, FSC and SSC data are acquired for the sample as well as fluorescence data for the fluorochrome-labeled antibody combinations as follows:

| PARAMETER | ANTIBODIES | FLUOR |
|---|---|---|
| FL1 | CD3 + CD4 | FITC |
| FL2 | CD8 + CD56 | PE |
| FL3 | CD45 | PerCP |

A single aliquot of blood is stained and lysed without washing and analyzed on the flow cytometer, triggering on CD45 PerCP fluorescence as previously described. B cells are presumptively identified as CD8-, CD56-, CD3-, CD4- cells (i.e., in the lower left area of a dotplot). NK cells are identified as CD8+, CD56+, CD3-, CD4- cells (i.e., in the upper left area of a dotplot). NK cells may also be identified by adding CD16 to CD8 + 56, as a small subset of NK cells are CD16+, CD56+. Cytotoxic suppressor T cells are identified as CD8+, CD56-, CD3+, CD4- cells (i.e., in the upper right area of a dotplot). Helper T cells are identified as CD8-, CD56-, CD3+, CD4+ cells (i.e., in the lower right area of a dotplot).

A two or three parameter gate may be set for the lymphocytes as previously described using any manual or automated means known in the art. It is preferred, however, that an autoclustering method capable of identifying subpopulations in multi-dimensional or N-dimensional space be used to automatically assign cluster classifications to the multi-parameter events recorded during data acquisition. Such an autoclustering method defined in claim 4 and is disclosed in PCT Application US 92/07291, filed August 28, 1992, (WO-A-9305478 published 18.03.93). The gravitational attractor described consists of a geometric boundary surface of fixed size, shape and orientation but with variable position. It is a computational engine by which the boundary surface positions itself optimally to enclose a cluster of multi-parameter events. Classification of events in the listmode data acquired is a two-step process in which 1) the datastream is analyzed for purposes of centering each attractor's boundary surface on the statistical center-of-mass of the data cluster it is intending to classify, and 2) locking each attractor's boundary in place and testing incoming datastream events against the boundaries for inclusion vs. exclusion decision-making. Such autoclustering methods are preferred over manual methods and previously known autoclustering methods for data analysis because they 1) allow analysis of data in real-time, 2) are tolerant of between-sample drift in the center-of-mass value of a data cluster due to changes in instrumentation, sample preparation and intrinsic sample variation, 3) are stable in cases where clusters are missing and 4) allow continuous process quality assurance during time-consuming rare-event assays. Using the inventive lysing, no-wash methodology with a CD45-PerCP trigger and "Attractors" software as described in PCT Application US 92/07291, the practitioner can develop and analyze 100% of the lymphocytes without contamination by debris, platelets or myeloid cells.

### EXAMPLE 1

### NO WASH SAMPLE ANALYSIS WITH 2-PARAMETER GATING

A whole blood sample (100 µL) was mixed with 0.25 µg HLe-1-PerCP antibody, 0.1 µg Leu-4-FITC antibody and 0.25 µg Leu-11+19-PE antibody (Becton Dickinson Immunocytometry Systems, San Jose, California). The PE conjugates were processed by cation exchange and sizing by gel filtration to remove all detectable free PE and multiples (2:1 and 1:2 conjugates) so that greater than 90% of the product had a PE:Ig ratio to 1:1. This process improved performance of the no-wash reagents, possibly due to the tendency of free PE to stick nonspecifically to platelets which are by definition present in a no-wash sample. The inventive no-wash sample preparation required as much as 20-fold less PE for staining when a high proportion of 1:1 conjugates were used and resulted in reduced background fluorescence.

The mixture was incubated 15 min. at room temperature in the dark. Two ml of FACS Lysing Solution (Becton Dickinson Immunocytometry Systems) were added to the stained sample and it was incubated for 10 min. at room temperature in the dark. The lysed, labeled sample was then analyzed on a FACSCAN flow cytometer, triggering on FL3 (anti-HLe-1-PerCP) and acquiring fluorescence and light scatter data with CONSORT 32 software. Analysis of the acquired data was performed using FACSCAN Research, LYSYS II or SIMULSET software.

Fig. 2A-D show a comparison of lymphocyte gating on SSC vs. FL3 to lymphocyte gating on SSC vs. FSC (Fig 2A) for the unwashed, lysed sample. Gating on SSC vs. FL3 (Fig. 2B) significantly reduced the amount of debris included in the gate, as is shown in the plot of FL1 vs. FL2 for the gated leukocyte subpopulation (compare Fig 2C and 2D). Debris can be reduced as much as 50% using SSC vs. FL3 to establish the gate as compared to the traditional SSC/FSC gate. Triggering on FL3 allows acquisition of a greater number of analyzable cells in situations where a fixed number of events is being acquired since events which may be detected by scatter but are below the fluorescence threshold are not counted. Under certain experimental conditions, triggering on fluorescence may also improve the speed of data acquisition.

### EXAMPLE 2

### NO WASH SAMPLE ANALYSIS WITH 3-PARAMETER GATING

Whole blood samples for eleven subjects (four 100 µL aliquots for each subject) were prepared on the FACSPREP, staining with 20 µL of anti-HLE-1-PerCP, anti-Leu-M7-FITC and anti-Leu-M3-PE (Becton Dickinson Immunocytometry Systems). Samples were incubated for about 15 min. at room temperature in the dark. Following labeling, 2 ml. of FACS Lysing Solution was added to each sample. These samples were analyzed on the FACSCAN without removal of the lysing solution or centrifugation and washing. These aliquots were analyzed triggering on FL3 (PerCP) and the data acquired in LYSYS for three-color analysis, gating the lymphocyte subpopulation.

No-wash data was analyzed using (1) LYSYS II with the conventional FSC/SSC scatter gate (R1) and a manually set FL3 trigger, and (2) LYSYS II with a manually set FL3 trigger and a 3-parameter gate based on SSC vs. FSC (R1) and SSC vs. FL3 (R2);

For comparison, two additional 100 µL aliquots of each sample were prepared on the FACSPREP, staining with SIMULTEST reagent kits (Becton Dickinson Immunocytometry Systems) according to the manufacturer's instructions, including centrifuging and washing the samples after lysing. These aliquots were analyzed on the FACSCAN with conventional triggering on light scatter. Data was acquired in SIMULSET software (Becton Dickinson Immunocytometry Systems) for two-color analysis, gating the lymphocyte subpopulation. For one washed sample the gate was set automatically by the software and for the second the gate was set manually by the operator.

In the washed samples using SIMULTEST staining, SIMULSET software and a light scatter trigger the two gating methods failed to eliminate debris from the gated lymphocyte subpopulation (1-8% contamination). There was some improvement in the quality of the gate when the operator redefined the gates. These gated lymphocyte subpopulations were also contaminated with 0-1% monocytes and 1-8% granulocytes.

In contrast, in the no-wash aliquots triggering on FL3 was effective to eliminate debris from the lymphocyte gate to levels below the detection limits of the analysis system, regardless of the method of data acquisition and gating. Monocytes were also eliminated from the gate in the no-wash format, regardless of whether the scatter gate or the 3-parameter gate was applied. Contamination of the gated lymphocyte population by granulocytes was significantly reduced by 3-parameter gating (0.2% average contamination), whereas the light scatter lymphocyte gate included between 1% and 6% granulocytes (probably primarily basophils). The 3-parameter gating method, in contrast to conventional 2-parameter light scatter gating, allows the operator to include essentially 100% lymphocytes in the gate with, on the average, no debris, no monocytes and only 0.2% granulocytes.

3-parameter gating is illustrated in Figs. 3A-3D. R1 (Fig. 3A) and R2 (Fig. 3B) are established and the cells included in both are identified to form R3 (Fig. 3D). The improved purity of the 3-parameter gate can be seen by comparing Fig. 3D with Fig. 3C, which illustrates R1 alone (conventional SSC/FSC gate but with an FL3 trigger). Staining of the gated lymphocytes with anti-Leu-M3 and anti-Leu-M7 quantitates the comparative degree of contamination with myeloid cells using the two gating methods. As the Figures show, contamination of gated lymphocytes by myeloid cells is significantly reduced by 3-parameter gating.

### EXAMPLE 3

### ANALYSIS OF BLOOD SAMPLES CONTAINING ERYTHROCYTES

A blood sample was stained with anti-Leu-3a-PE, anti-Leu-2a-FITC and anti-HLe-PerCP, lysed with ammonium chloride, centrifuged and washed once with phosphate buffered saline. The sample was then split and red blood cells were added to one aliquot in sufficient quantity that about 70-80% of the events included in the gate were due to RBC's. The aliquots were analyzed on a FACSCAN flow cytometer and lymphocyte gates were set using SSC vs. FSC (Fig 4A and 4E) and SSC vs. FL3 (PerCP) (Fig 4B and 4F). FL1 and FL2 events were then plotted and counted for the gated lymphocytes.

In the aliquot without added red blood cells, there was essentially no difference in the percentage of Leu-3a and Leu-2a positive cells with the two gating methods (compare Fig. 4C and Fig. 4D). In the aliquot containing red blood cells, accurate Leu-3a positive and Leu-2a positive percentages were obtained using SSC vs. FL3 gating, but SSC vs. FSC gating produced highly inaccurate results (Fig. 4H and Fig. 4G).

These results demonstrate that the present gating method does not result in loss of any cell populations as compared to conventional scatter gating. In addition, the method provides a means for compensating for light scatter interference from red blood cells in unlysed blood samples and lysing reagents in no-wash blood sample preparation methods.

### EXAMPLE 4

### THREE-PART DIFFERENTIAL WITH CD4/CD8 SUBSETTING

A single aliquot of blood was simultaneously stained with anti-HLE-1-PerCP, anti-Leu-3-FITC, anti-Leu-4-FITC, anti-Leu-2-PE and anti-NCAM Leu-19-PE, and lysed without washing as previously described. The stained sample was analyzed on a FACSCAN, triggering on FerCP fluorescence. FSC, SSC and fluorescence data for each event detected were acquired and analyzed using attractors software to identify the lymphocyte cluster. Fig. 5A shows the lymphocyte subpopulation free of debris and myeloid cells after analysis using "Attractors" software. To identify Tₕₑₗₚₑᵣ, Tₛᵤₚᵣₑₛₛₒᵣ, B and NK cell subsets, dotplots of anti-Leu-3-FITC + anti-Leu-4-FITC (CD3 + CD4 FITC) vs. anti-Leu-2-PE + anti-NCAM Leu-19-PE (CD8 + CD56 PE) were generated (Fig. 5B) and the percent of total lymphocytes in each subset calculated.

## Claims

1. A method for identifying by flow cytometry a sub-population of cells of interest in a population of leukocytes in a blood sample, the method comprising in a single aliquot of the sample:
a) labelling the leukocytes with a leukocyte-specific fluorochrome-labelled primary antibody which binds to different extents to the cells of the sub-population of interest and to the cells of at least one additional sub-population;
b) lysing red blood cells in the sample by addition of a lysing reagent;
c) analysing the labelled leukocytes in the lysed sample on a flow cytometer without separation of the labelled leukocytes from the lysing reagent, the cytometer being set with a triggering threshold representative of a predetermined fluorescence intensity indicative of the presence of a primary antibody, such that all cells for which said threshold is exceeded, are analysed,
said analysing comprising
d) acquiring fluorescence and light scatter data for the labelled leukocytes; and
e) identifying the sub-population of cells of interest by a method including generating a first plot of a light scatter intensity parameter vs. antibody fluorescence intensity and selecting a gate area of said plot as said sub-population of cells of interest.

2. A method according to Claim 1, wherein said identifying step comprises:
e₁) establishing a second plot of side light scatter intensity vs. forward light scatter intensity;
e₂) establishing as said first plot, a plot of side light scatter intensity vs. primary antibody fluorescence intensity; and
e₃) establishing a third plot by selecting the cells included in both the first and second plots, thereby identifying the cells of the sub-population of interest.

3. A method according to Claim 1 or 2, wherein the leukocytes are labelled with at least one fluorochrome-labelled secondary antibody which is specific for and binds to the cells of a subset of the sub-population of interest, primary antibody fluorescence being distinguishable from secondary antibody fluorescence by flow cytometry.

4. A method according to Claim 1, wherein said identifying step comprises autoclustering the cells of the sub-population, using a method comprising:
i) for each sub-population expected in the sample, fixing a geometric boundary surface so as to confer membership in the sub-population that is fixed in shape, size and orientation, but not position prior to autoclustering;
ii) setting a seed location and radius for each sub-population;
iii) transforming a vector for each cell analysed into a co-ordinate system, wherein the vector comprises values for each parameter acquired;
iv) summing each vector to calculate a vector mean if the proximity of that vector is less than the radius distance from the centre location of the vector;
v) after a predetermined number of vectors are added to the vector sum to calculate the vector mean calculating the centre location as the vector mean;
vi) repeating steps iii)-v) until a predetermined number of vectors have been included in the calculation of the vector mean;
vii) establishing a final geometric boundary based upon the last centre location calculated; and
viii) comparing all subsequent cell vectors against the final boundary for inclusion within or exclusion outside the boundary, thereby identifying the sub-population of interest.

5. A method according to Claim 4, further comprising labelling the leukocytes with at least one secondary fluorochrome-labelled antibody specific for the cells of a subset of the sub-population, the primary antibody fluorescence being distinguishable from secondary antibody fluorescence by flow cytometry and identifying the cells of the subset by analysis of secondary antibody fluorescence of the cells of the sub-population.

6. The method of Claim 4, wherein the cells of the leukocyte population are labelled with a CD45-PerCP antibody and the cells of the sub-population are labelled with CD3-FITC, CD4-FITC, CD8-PE and CD56-PE.

7. The method of Claim 5, wherein T-helper, T-suppressor, B and NK cells are identified by analysis of FITC and PE fluorescence of CD45-PerCP positive lymphocytes.

8. A method for identifying by flow cytometry a sub-population of cells of interest in a population of leukocytes in a blood sample according to claim 1, wherein:
- in step a) the cells of the population are labelled with an anti-leukocyte antibody conjugated to a first fluorochrome (FL1), with an anti-monocyte antibody conjugated to a separately detectable second fluorochrome (FL2) and with said anti-leukocyte primary antibody conjugated to a third fluorochrome (FL3) which can be differentiated from the first and second fluorochrome;
- in step d) fluorescence data (FL1 and FL2) and light scatter data are acquired for the labelled leukocytes;
f) the method further including the steps of labelling the cells of the population in a second aliquot of the sample with said primary anti-leukocyte antibody conjugated to said third fluorochrome (FL3);
g) analysing the labelled second aliquot on a flow cytometer, without separation of the labelled leukocytes from the lysing reagent, the cytometer being set with a triggering threshold representative of a predetermined fluorescence intensity indicative of the presence of said primary antibody, such that cells for which said threshold is exceeded, are analysed, said analysing comprising acquiring fluorescence and light scatter data for the cells of the labelled population in said second aliquot; and
h) applying the gate area selected in step e) to the data acquired for the second aliquot, thereby identifying the cells of the sub-population in the second aliquot.

## Patentansprüche

1. Verfahren zur Bestimmung einer Subpopulation von interessierenden Zellen in einer Leukozyten-Population in einer Blutprobe mittels Durchflußzytometrie, welches Verfahren bei einem einzelnen Aliquot der Probe umfaßt:
a) Markierung der Leukozyten mit einem Leukozyt-spezifischen, Fluorochrommarkierten primären Antikörper, der sich in unterschiedlichem Maß an die Zellen der interessierenden Subpopulation und an die Zellen mindestens einer weiteren Subpopulation bindet;
b) Lyse der roten Blutzellen in der Probe durch Zugabe eines Lyse-Reagens;
c) Analyse der markierten Leukozyten in der lysierten Probe in einem Durchflußzytometer ohne Abtrennung der markierten Leukozyten vom Lyse-Reagens, welches Zytometer auf eine Aktivierungsschwelle eingestellt ist, die für eine vorbestimmte Fluoreszenzintensität, welche auf das Vorhandensein von primären Antikörpern schließen läßt, repräsentativ ist, so daß alle Zellen analysiert werden, bei denen die Schwelle überschritten wird, wobei die Analyse umfaßt:
d) Erfassung von Fluoreszenz- und Lichtstreuungsdaten für die markierten Leukozyten; und
e) Bestimmung der Subpopulation der interessierenden Zellen mittels eines Verfahrens, welches das Erzeugen einer ersten Graphik eines Lichtstreuungsintensität-Parameters gegen die Antikörper-Fluoreszenzintensität und das Auswählen einer Gate-Fläche der Graphik als Subpopulation der interessierenden Zellen miteinschließt.

2. Verfahren nach Anspruch 1, wobei der Bestimmungsschritt umfaßt:
e₁) Aufnahme einer zweiten Graphik von Seitenlichtstreuungsintensität gegen Lichtstreuungsintensität von vorwärts einfallendem Licht;
e₂) Aufnahme einer Graphik von Seitenlichtstreuungsintensität gegen die Fluoreszenzintensität primärer Antikörper als besagte erste Graphik; und
e₃) Aufnahme einer dritten Graphik durch Auswählen der sowohl in der ersten als auch in der zweiten Graphik enthaltenen Zellen, wodurch die Zellen der interessierenden Subpopulation bestimmt werden.

3. Verfahren nach Anspruch 1 oder 2, wobei die Leukozyten mit mindestens einem Fluorochrom-markierten sekundären Antikörper markiert werden, der für die Zellen einer Teilmenge der interessierenden Subpopulation spezifisch ist und sich an diese bindet, und wobei die Fluoreszenz der primären Antikörper mittels Durchflußzytometrie von der Fluoreszenz der sekundären Antikörper unterscheidbar ist.

4. Verfahren nach Anspruch 1, wobei der Bestimmungsschritt ein Autoclustering der Zellen der Subpopulation unter Verwendung eines Verfahrens umfaßt, welches:
i) das Festlegen einer geometrischen Grenzfläche, aber keiner Position, für jede in der Probe erwartete Subpopulation vor dem Autoclustering, um einer in Form, Größe und Ausrichtung fixierten Subpopulation Zusammengehörigkeit zu verleihen; ii) das Festsetzen eines Keimorts und -radius für jede Subpopulation;
iii) das Übertragen eines Vektors für jede analysierte Zelle in ein Koordinatensystem, in welchem der Vektor aus Werten für jeden erfaßten Parameter besteht;
iv) das Summieren jedes Vektors zur Berechnung eines Vektormittels, wenn die Nähe dieses Vektors geringer als der Radiusabstand von der zentralen Stelle des Vektors ist;
v) das Berechnen der zentralen Stelle als Vektormittel, nachdem eine im voraus festgelegte Anzahl von Vektoren zur Vektorsumme zwecks Berechnung des Vektormittels hinzugefügt wurde;
vi) das Wiederholen der Schritte iii)-v), bis eine im voraus festgelegte Anzahl von Vektoren in die Berechnung des Vektormittels miteinbezogen wurde;
vii) das Einführen einer endgültigen geometrischen Grenze basierend auf der zuletzt brechneten Stelle; und
viii) das Vergleichen aller späteren Zellvektoren mit der endgültigen Grenze zwecks Aufnahme innerhalb oder Ausschlusses außerhalb der Grenze, wodurch die interessierende Subpopulation bestimmt wird,
umfaßt.

5. Verfahren nach Anspruch 4, welches weiters das Markieren der Leukozyten mit mindestens einem sekundären Fluorochrom-markierten Antikörper, der für die Zellen einer Teilmenge der Subpopulation spezifisch ist, wobei die Fluoreszenz der primären Antikörper von der Fluoreszenz der sekundären Antikörper mittels Durchflußzytometrie unterscheidbar ist, und das Bestimmen der Zellen der Teilmenge durch Analyse der Fluoreszenz der sekundären Antikörper der Zellen der Subpopulation umfaßt.

6. Verfahren nach Anspruch 4, wobei die Zellen der Leukozyten-Population mit einem CD45-PerCP Antikörper und die Zellen der Subpopulation mit CD3-FITC, CD4-FITC, CD8-PE und CD56-PE markiert werden.

7. Verfahren nach Anspruch 5, wobei T-Helfer-, T-Hemm-, B- und NK-Zellen mittels Analyse der FITC- und PE-Fluoreszenz der CD45-PerCP-positiven Lymphozyten bestimmt werden.

8. Verfahren zur Bestimmung einer Subpopulation von interessierenden Zellen in einer Leukozyten-Population in einer Blutprobe mittels Durchflußzytometrie nach Anspruch 1, wobei:
- in Stufe a) die Zellen der Population mit einem an einen ersten Fluorochrom (FL1) konjugierten anti-Leukozyt-Antikörper, mit einem an einen getrennt detektierbaren zweiten Fluorochrom (FL2) konjugierten anti-Monocyt-Antikörper und mit besagtem an einen dritten Fluorochrom (FL3) konjugierten primären anti-Leukozyt-Antikörper markiert werden, welcher dritte Fluorochrom vom ersten und zweiten Fluorochrom unterschieden werden kann;
- in Schritt d) Fluoreszenzdaten (FL1 und FL2) und Lichtstreuungsdaten für die markierten Leukozyten erfaßt werden;
f) das Verfahren weiters die Schritte des Markierens der Zellen der Population in einem zweiten Aliquot der Probe mit besagtem, an den dritten Fluorochrom (FL3) konjugierten primären anti-Leukozyt-Antikörper;
g) des Analysierens des markierten zweiten Aliquots in einem Durchflußzytometer ohne Abtrennung der markierten Leukozyten vom Lyse-Reagens, welches Zytometer auf eine Aktivierungsschwelle eingestellt ist, die für eine vorbestimmte Fluoreszenzintensität, welche auf das Vorhandensein des primären Antikörpers schließen läßt, repräsentativ ist, so daß Zellen analysiert werden, bei denen die Schwelle überschritten wird, wobei das Analysieren die Erfassung von Fluoreszenz- und Lichtstreuungsdaten für die Zellen der markierten Population im zweiten Aliquot umfaßt; und
h) des Anwendens der in Schritt e) ausgewählten Gate-Fläche auf die für das zweite Aliquot erworbenen Daten, umfaßt, wodurch die Zellen der Subpopulation im zweiten Aliquot bestimmt werden.

## Revendications

1. Méthode d'identification par cytométrie de flux d'une sous-population de cellules intéressantes dans une population de leucocytes dans un échantillon de sang, la méthode comprenant, dans une fraction aliquote individuelle de l'échantillon, les étapes consistant :
a) à marquer les leucocytes avec un anticorps primaire marqué par un fluorochrome spécifique des leucocytes qui se lie à des degrés différents aux cellules de la sous-population intéressante et aux cellules d'au moins une autre sous-population ;
b) à lyser les globules rouges dans l'échantillon par addition d'un réactif de lyse ;
c) à analyser les leucocytes marqués dans l'échantillon lysé dans un cytomètre de flux sans séparer les leucocytes marqués du réactif de lyse, le cytomètre étant réglé avec un seuil de déclenchement représentatif d'une intensité de fluorescence prédéterminée indicatrice de la présence d'un anticorps primaire, tel que toutes les cellules pour lesquelles ledit seuil est dépassé soient analysées,
ladite analyse comprenant les étapes consistant
d) à acquérir des données de fluorescence et de diffusion de la lumière pour les leucocytes marqués ; et,
e) à identifier la sous-population de cellules intéressantes par une méthode incluant la production d'une première courbe d'un paramètre d'intensité de diffusion de la lumière vs. intensité de fluorescence de l'anticorps et à sélectionner une zone portail de ladite courbe comme sous-population de cellules intéressantes.

2. Méthode selon la revendication 1, dans laquelle ladite étape d'identification comprend les étapes consistant :
e₁) à établir une seconde courbe d'intensité de diffusion de la lumière latérale vs. intensité de diffusion de la lumière vers l'avant ;
e₂) à établir comme dite première courbe, une courbe d'intensité de diffusion de la lumière latérale vs. intensité de fluorescence de l'anticorps primaire, et e₃) à établir une troisième courbe en sélectionnant les cellules inclues à la fois dans la première et la seconde courbes, puis en identifiant les cellules de la sous-population intéressantes.

3. Méthode selon la revendication 1 ou 2, selon laquelle les leucocytes sont marqués par au moins un anticorps secondaire marqué par un fluorochrome qui est spécifique des cellules et se lie aux cellules d'un sous ensemble de la sous-population intéressante, la fluorescence de l'anticorps primaire pouvant être distinguée de la fluorescence de l'anticorps secondaire par cytométrie de flux.

4. Méthode selon la revendication 1, dans laquelle ladite étape d'identification comprend l'auto-regroupement des cellules de la sous-population, en utilisant une méthode comprenant les étapes consistant :
i) pour chaque sous-population attendue dans l'échantillon, à fixer une surface de frontière géométrique de façon à conférer un statut de membre de la sous-population, dont la forme, la taille et l'orientation sont fixées, mais non la position avant l'auto-regroupement ;
ii) à établir une localisation de germe et un rayon pour chaque sous-population ;
iii) à transformer un vecteur pour chaque cellule analysée dans un système coordonné, le vecteur comprenant des valeurs pour chaque paramètre acquis ;
iv) à additionner chaque vecteur pour calculer une moyenne de vecteurs si la proximité de ce vecteur est inférieure à la distance du rayon par rapport à la localisation du centre du vecteur ;
v) après avoir ajouté un nombre prédéterminé des vecteurs à la somme des vecteurs pour calculer la moyenne des vecteurs, à calculer la localisation du centre comme moyenne de vecteurs ;
vi) à répéter les étapes iii)-v) jusqu'à ce qu'un nombre prédéterminé de vecteurs ait été inclus dans le calcul de la moyenne des vecteurs ;
vii) à établir une frontière géométrique finale basée sur la dernière localisation de centre calculée ; et
viii) à comparer tous les vecteurs de cellules suivants par rapport à la frontière finale pour une inclusion à l'intérieur de la frontière ou une exclusion à l'extérieur de la frontière, en identifiant ainsi la sous-population intéressante.

5. Méthode selon la revendication 4, comprenant en outre le marquage des leucocytes par au moins un anticorps marqué par un fluorochrome secondaire spécifique des cellules d'un sous-ensemble de la sous-population, la fluorescence de l'anticorps primaire pouvant être distinguée de la fluorescence de l'anticorps secondaire par cytométrie de flux, et l'identification des cellules du sous-ensemble par analyse de la fluorescence de l'anticorps secondaire des cellules de la sous-population.

6. Méthode de la revendication 4, dans laquelle les cellules de la population de leucocytes sont marquées par un anticorps CD45-PerCP et les cellules de la sous-population sont marquées par CD3-FITC, CD4-FITC, CD8-PE et CD56-PE.

7. Méthode de la revendication 5, dans laquelle des lymphocytes T auxiliaires, des lymphocytes T suppresseurs, des lymphocytes B et des cellules NK sont identifiés par analyse de la fluorescence FITC et PE de lymphocytes positifs pour CD45-PerCP.

8. Méthode d'identification par cytométrie de flux d'une sous-population de cellules intéressantes dans une population de leucocytes dans un échantillon de sang selon la revendication 1, dans laquelle :
- dans l'étape a), les cellules de la population sont marquées par un anticorps anti-leucocyte conjugué à un premier fluorochrome (FL1), avec un anticorps anti-monocyte conjugué à un second fluorochrome séparément détectable (FL2) et ledit anticorps primaire anti-leucocyte étant conjugué à un troisième fluorochrome (FL3) qui peut être différencié du premier et du second fluorochrome ;
- dans l'étape d), les données de fluorescence (FL1 et FL2) et les données de diffusion de la lumière sont acquises pour les leucocytes marqués ;
f) la méthode incluant en outre les étapes consistant à marquer les cellules de la population dans une seconde fraction aliquote de l'échantillon avec ledit anticorps anti-leucocyte primaire conjugué audit troisième fluorochrome (FL3) ;
g) à analyser la seconde fraction aliquote marquée dans un cytomètre de flux, sans séparer les leucocytes marqués du réactif de lyse, le cytomètre étant réglé avec un seuil de déclenchement représentatif d'une intensité de fluorescence prédéterminée indicatrice de la présence dudit anticorps primaire, de telle sorte que les cellules pour lesquelles ledit seuil est dépassé soient analysées, ladite analyse comprenant l'acquisition des données de fluorescence et de diffusion de la lumière pour les cellules de la population marquée dans ladite seconde fraction aliquote ; et
h) à appliquer la zone portail sélectionnée dans l'étape e) aux données acquises pour la seconde fraction aliquote, en identifiant ainsi les cellules de la sous-population dans la seconde fraction aliquote.
